# EUROPÄISCHE PATENTSCHRIFT

(11) **EP 1 949 918 B1**
(45) Veröffentlichungstag und Bekanntmachung des Hinweises auf die Patenterteilung: **08.08.2012**
(21) Anmeldenummer: 07024028.8
(22) Anmeldetag: 12.12.2007
(51) Int. Cl.: A61L 24/00, A61L 24/06, A61L 27/16, A61L 27/54

(54) **Revisions-Polymethylmethacrylat-Knochenzement**
Revision polymethyl methacrylate bone cement
Ciment osseux de révision en polyméthylméthacrylate

(30) Priorität: 26.01.2007 DE 102007004968
(43) Veröffentlichungstag der Anmeldung: 30.07.2008
(73) Patentinhaber: Heraeus Kulzer GmbH, 63450 Hanau (DE)
(72) Erfinder: Vogt, Sebastian, 99092 Erfurt (DE); Kühn, Klaus-Dieter, 35041 Marburg-Einhausen (DE); Büchner, Hubert, 64354 Reinheim (DE)
(74) Vertreter: Kühn, Hans-Christian

(56) Entgegenhaltungen:
- EP-A1- 0 676 408
- DE-A1-102004 049 121

## Beschreibung

Gegenstand der Erfindung ist ein Revisions-Polymethylmethactylat-Knochenzement (Revisions-PMMA-Knochenzement).

Gelenkendoprothesen haben gegenwärtig eine Standzeit von mehreren Jahren, z.B. bei zementierten Hüftgelenkprothesen im Durchschnitt 10-15 Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich keine mikrobiellen Keime nachweisen. Die Ursachen der aseptischen Lockerungen können vielfältig sein. Häufig sind aseptische Lockerungen auf den Abrieb an den Gleitflächen der Gelenkendoprothesen zurückzuführen. Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich mit sehr hohen Kosten verbunden ist. Sowohl bei der aseptischen als auch der septischen Lockerung wird üblicherweise eine Revision vorgenommen. Man unterscheidet dabei die einzeitige und die zweizeitige Revision. Bei septischen Lockerungen können neben zementfreien Revisionsprothesen auch zementierte Revisionsprothesen zusammen mit PMMA-Knochenzementen sowohl bei der einzeitigen als auch bei der zweizeitigen Revision Verwendung finden. Diese PMMA-Knochenzemente sollten dabei, nach erfolgtem Nachweis der vorliegenden Keime und nach Anfertigung eines Antibiogramms, mit geeigneten Antibiotika ausgerüstet sein. Üblich ist dabei die Verwendung von Kombinationen von zwei oder drei unterschiedlichen Antibiotika, die möglichst unterschiedliche Wirkungsmechanismen gegenüber den bestimmten Mikroorganismen aufweisen sollten. Diese Antibiotika werden in der Klinik von Chirurgen oder von Apothekem konventionellen PMMA-Knochenzementen unter weitgehend aseptischen Bedingungen beigemischt (H. Breithaupt: Lokale Antibiotikatherapie. In: Septische Knochenchirurgie. Hrsg. R. Schnettler, H.-U. Steinau, Georg Thieme Verlag Stuttgart New York, 2004). Es ist dabei wünschenswert, dass eine möglichst hohe initiale Freisetzung der Antibiotika aus dem PMMA-Knochenzement nach der Implantation an der Grenzfläche Knochenzement/Knochen erreicht wird.

Antibiotika-modifizierte PMMA-Knochenzemente (Polymethylmethacrylat-Knochenzemente) sind seit den sechziger Jahren des 20. Jahrhunderts basierend auf den Arbeiten von H. W. Buchholz und der Firma Kulzer bekannt (W. Ege, K.-D. Kühn: Industrial development of bone cement - 25 years of experience. In: Bone Cement and Cementing Technique. Eds. G. H. I. M. Walenkamp, D. W. Murray, Springer Verlag Heidelberg, 2001, in press; H. W. Buchholz, E. Engelbrecht: Über die Depotwirkung einiger Antibiotika beim Vermischen mit dem Kunstharz Palacos. Chirurg 41 (1970) 511-515). Diese PMMA-Zemente haben eine breite Akzeptanz gefunden und werden in großem Umfang zur Fixierung von Endoprothesen eingesetzt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Das in den üblichen PMMA-Knochenzementen integrierte Antibiotikum wird nach der Implantation mehr oder weniger schnell an der Grenzfläche KnochenzementlKnochen lokal freigesetzt und soll dort die bakterielle Besiedlung verhindern. Angestrebt wird eine möglichst hohe initiale Freisetzung, so dass die minimale Hemmkonzentration (MHK) des eingesetzten Antibiotikums gegenüber den klinisch relevanten Keimen an der Grenzfläche Knochenzement/Knochen sicher erreicht und überschritten wird. Dadurch kann die Grenzfläche zwischen dem Knochen und dem PMMA-Knochenzement vor mikrobieller Besiedlung über einen Zeitraum von einigen Tagen nach der Implantation geschützt werden.

Im DE102004049121 (A1) wird ein PMMA-Knochenzement vorgeschlagen, der dadurch charakterisiert ist, dass in der Pulverkomponente 0,1-5,0 Masseprozent in Wasser lösliche, glasartige Antibiotikum-/Antiblotika-Granula mit einem Partikeldurchmesser im Bereich von 63-900 µm enthalten sind, die aus miteinander verbundenen, glasartigen Antibiotikum-/Antibiotika-Primärparfikein aufgebaut sind, welche einen Partikeldurchmesser im Bereich von 1-70 µm haben.

Die Aufgabe der Erfindung besteht darin, einen Revisions-PMMA-Knochenzement zu entwickeln, der mit zwei oder mehreren Antibiotika ausgerüstet ist, der nach lmplantation eine hohe initale Freisetzung aller im Zement enthaltenden Antibiotika gewährleistet.

Der Erfindung liegt der überraschende Befund zu Grunde, dass PMMA-Knochenzemente, die zwei oder mehrere granuläre Antibiotika enthalten, eine hohe initiale Antibiotika-Freisetzung zeigen, wenn die Komgrößenverteilung der eingesetzten Antibiotika annähernd gleich ist. Unter dem Begriff granuläre Antibiotika werden im Folgenden bei Raumtemperatur im festen Aggregatzustand vorliegende Antibiotika-Kömchen verstanden, die regelmäßig oder unregelmäßig geformt sind. Die Antibiotika-Kömchen können sowohl kristallin als auch amorph sein. Es ist auch möglich, dass die Antibiotika-Kömchen teilkristallin sind.

Die Aufgabe wurde durch die Entwicklung eines Revisions-PMMA-Knochenzements gelöst, In diesem Revisions-PMMA-Knochenzement mit Pulver- und Flüssig-Komponente sind in der Pulverkomponente zwei oder mehr granuläre Antibiotika enthalten, die in der Komgrößenverteilung darin übereinstimmen, dass jeweils die Haupt-Siebfraktion der einzelnen Antibiotika in ein- und demselben Komgrößenbereich liegt.

Vorzugsweise umfasst die jeweilige Haupt-Siebfraktion der Antibiotika jeweils mindestens 50 Gewichtsprozent des betreffenden Antibiotikums.
Der Komgrößenbereich der Hauptsiebfraktion der einzelnen Antibiotika beträgt insbesondere 100 bis 250 µm oder 150 bis 250 µm.

Die Komgrößenverteilung stimmt überein, wenn die Siebfraktionen der einzelnen Antibiotika annähernd gleich sind. Es genügt aber, wenn die Haupt-Siebfraktionen der Antibiotika die gleiche Komgröße haben. Haupt-Siebfraktionen sind insbesondere diejenigen von jeweils mindestens 50 Gewichtsprozent jedes Antibiotikums.

Unter der Pulverkomponente des PMMA-Knochenzementes wird ein Gemisch aus mindestens einem pulverförmigen Polymethylmethacrylat oder einem Kopolymer, das aus Methylmethacrylat und Methylacrylat aufgebaut ist, einem pulverförmigen Röntgenopaker, wie Zirkoniumdioxid und/oder Bariumsulfat, und einem radikalischen Initiator, wie Dibenzoylperoxid, verstanden. Gegebenenfalls sind Bestandteile der Pulverkomponente mit einem pharmazeutisch akzeptablen Farbstoff eingefärbt. Die Pulverkomponente ergibt nach Vermischung mit der flüssigen Komponente, die aus Methylmethacrylat (MMA), in dem ein radikalischer Aktivator, wie N,N-Dimethyl-p-toluidin, gelöst ist, aufgebaut ist, einen plastisch verformbaren Teig, der nach wenigen Minuten selbständig durch die einsetzende radikalische Polymerisation des Methylmethacrylates aushärtet.

Der erfindungsgemäß hergestellte PMMA-Knochenzement zeigte unter in vitro-Bedingungen bei 37 °C eine sehr hohe Antibiotikum-Freisetzung.

Die granulären Antibiotika bestehen z. B. aus mindestens einem Vertreter aus den Gruppen der Aminoglykosid-Antibiotika, der Lincosamid-Antibiotika, der Fluorchinolon-Antibiotika, der Glykopeptid-Antibiotika und der Nitroimidazole. Die antimikrobiell wirksamen Chemotherapeutika aus der Gruppe der Nitroimidazole werden vereinfachend auch als Antibiotika verstanden. Diese Chemotherapeutika wirken hauptsächlich bakterizid gegen anaerobe Keime und gegen Protozoen.

Es ist im Rahmen der Erfindung bevorzugt, dass die granulären Antibiotika aus Gentamicinsulfat, Gentamicinhydrochlorid, Amikacinsulfat, Amikacinhydrochlorid, Tobramycinsulfat, Tobramycinhydrochiorid, Clindamycinhydrochlorid, Lincosaminhydrochlorid, Moxifloxacin, Ciprofloxacin, Teicoplanin, Vancomycin, Ramoplanin, Dalbavancin, Daptomycin, Tigecyglin, Metronidazol, Tinidazol und Omidazol bestehen. Neben diesen in Wasser löslichen Antibiotikasalzen und Antibiotika können auch in Wasser gering lösliche Salzformen der Antibiotika, wie beispielsweise Flavonphosphate, Palmitate, Myristate und Laurate, verwendet werden oder zusätzlich enthalten sein. Weiterhin ist es auch möglich, Antibiotika aus der Gruppe der Oxazolidone, wie Linezolid, zu verwenden oder zuzusetzen.

Vorteilhaft ist weiterhin, wenn die granulären Antibiotika gegebenenfalls als Hilfsstoffe zusätzlich Polyvinylpyrrolidon und/oder Polyethylenglykol und/oder Polyethylenoxid und/oder Maltose und/oder Sorbitol und/oder Mannitol enthalten können. Ebenfalls im Rahmen der Erfindung ist, wenn die granulären Antibiotika durch andere toxikologisch akzeptable Polymere, wie Gelatine, Kollagen und Dextran, stabilisiert sind. Im weiteren Sinne der Erfindung können auch granuläre Antibiotika enthalten sein, die mit adhäsiven Hilfsstoffen zu Antibiotika-Granula mit Partikelgrößen im Bereich von 63-900 µm verklebt oder verkittet wurden, wie in DE102004049121 A1 beschrieben.

Die Erfindung wird anhand des folgenden Beispiels erläutert, ohne jedoch die Erfindung einzuschränken. Teile und Prozentangaben beziehen sich wie in der übrigen Beschreibung auf das Gewicht, sofern nicht anders angegeben.

### Beispiel 1:

Zur Prüfung des erfindungsgemäßen PMMA-Knochenzementes wurden Freisetzungsuntersuchungen an Probekörpern durchgeführt. Die Präparation der Probekörper erfolgte in der Weise, dass zuerst jeweils 40,0 g der Pulverkomponente des Knochenzementes Palacos R mit

| | |
|---|---|
| Variante a) | 0,80 g Gentamicinsulfat (entspricht 0,50 g Gentamicinbase; Siebfraktionen: 2 % < 63 µm; 3 % 63-100 µm; 93 % 100 -250 µm; 2 % > 250 µm) + 2,17 g Vancomycinhydrochlorid (entspricht 2,00 g Vancomycinbase; Siebfraktionen: 43 % < 63 µm; 62 % 63-100 µm; 5 % 100 -250 µm; 0 % > µm) |
| Variante b) | 0,80 g Gentamicinsulfat (entspricht 0,50 g Gentamicinbase; Siebfraktionen: 2 % < 63 µm; 3 % 63-100 µm; 93 % 100 -250 µm; 2 % > 250 µm) + 2,17 g Vancomycinhydrochlorid (entspricht 2,00 g Vancomycinbase; Siebfraktionen: 3 % < 63 µm; 25 % 63-100 µm; 55 % 100 -250 µm; 19 % > 250 µm) |

vermischt wurde. Danach wurden diese modifizierten Pulverkomponenten mit jeweils 20,0 g der Monomerkomponente vermischt. Es bildete sich ein grüner Teig, der in Hohlformen gestrichen wurde und dort nach wenigen Minuten aushärtete. Die entstandenen zylinderförmigen Probekörper hatten eine Höhe von 1 cm und einen Durchmesser von 2,5 cm. Es wurden jeweils 5 Probekörper pro Zementvariante hergestellt. Die Probekörper wurden separat in jeweils 20 ml destilliertem Wasser bei 37 °C eingelagert. Täglich wurde das Feisetzungsmedium vollständig entnommen und darin die freigesetzte Gentamicinmenge bestimmt. Die Probekörper wurden dann wieder in jeweils 20 ml frischem destillierten Wasser bei 37 °C gelagert. Die Bestimmung des freigesetzten Gentamicins und des freigesetzten Vancomycins erfolgte mit einem TDX-Analyser der Firma Abott. Die jeweils freigesetzte Masse an Gentamicinbase und Vancomycinbase wurde pro Gramm Probekörper in der nachfolgenden Tabelle in Abhängigkeit von der Lagerungszeit der Probekörper im Freisetzungsmedium angegeben.

| Lagerungszeit [d] | | Freigesetzte Antibiotika | | |
|---|---|---|---|---|
| | | 1 d | 3 d | 5 d |
| Variante 1 | Gentamicin | 197 µg/g | 14 µg/g | 7 µg/g |
| | Vancomycin | 366 µg/g | 12 µg/g | 6 µg/g |
| Variante 2 | Gentamicin | 276 µg/g | 11 µg/g | 14 µg/g |
| | Vancomycin | 630 µg/g | 26 µg/g | 19 µg/g |

## Patentansprüche

1. Revisions-PMMA-Knochenzement mit Pulver- und Flüssig-Komponente, **dadurch gekennzeichnet, dass** in der Pulverkomponente zwei oder mehr granuläre Antibiotika enthalten sind, die in der Korngrößenverteilung darin übereinstimmen, dass jeweils die Haupt-Siebfraktion der einzelnen Antibiotika in ein- und demselben Korngrößenbereich liegt, wobei die Haupt-Siebfraktion der Antibiotika jeweils mindestens 50 Gewichtsprozent jedes Antibiotikums umfasst und wobei der Korngrößenbereich der Hauptsiebfraktion der einzelnen Antibiotika 100 - 250 µm beträgt.

2. Revisions-PMMA-Knochenzement nach Anspruch 1, **dadurch gekennzeichnet, dass** der Korngrößenbereich der Hauptsiebfraktion der einzelnen Antibiotika 150 - 250 µm beträgt.

3. Revisions-PMMA-Knochenzement nach Anspruch 1 oder 2 bei dem die granulären Antibiotika aus einem oder mehr Mitgliedern der Gruppe Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid, Moxifloxacin, Ciprofloxacin, Teicoplanin, Vancomycin, Ramoplanin, Dalbavancin, Daptomycin, Tigecyglin, Metronidazol, Tinidazol und Omidazol bestehen.

4. Revisions-PMMA-Knochenzement nach mindestens einem der vorstehenden Ansprüche, wobei als granuläre Antibiotika solche aus der Gruppe der Oxazolidone verwendet werden.

5. Revisions-PMMA-Knochenzement nach mindestens einem der vorstehenden Ansprüche, wobei die granuläre Antibiotika Komponente zusätzlich mindestens einen Hilfsstoff aus der Gruppe Polyvinylpyrrolidon, Polyethylenglykol, Polyethylenoxid, Maltose, Sorbitol und Mannitol enthält.

6. Revisions-PMMA-Knochenzement nach mindestens einem der vorstehenden Ansprüche, wobei die granuläre Antibiotika-Komponente zusätzlich mindestens einen Hilfsstoff aus der Gruppe der toxikologisch akzeptablen Polymere Gelatine, Kollagen und Dextran enthält.

7. Revisions-PMMA-Knochenzement nach mindestens einem der vorstehenden Ansprüche, wobei zumindest ein Teil der granulären Antibiotika mit adhäsiven Hilfsstoffen zu Antibiotika-Granula mit Partikelgrößen im Bereich von 63-900 µm verklebt oder verkittet sind.

## Claims

1. Revision PMMA bone cement comprising powder and liquid component, **characterised in that** the powder component contains two or more granular antibiotics, which have the same grain size distribution in terms of the grain size range of the individual antibiotics in the main sieve fraction of each being the same, whereby the main sieve fraction of the antibiotics each comprises at least 50 % by weight of each antibiotic and whereby the grain size range of the main sieve fraction of the individual antibiotics is 100 - 250 µm.

2. Revision PMMA bone cement according to claim 1, **characterised in that** the grain size range of the main sieve fraction of the individual antibiotics is 150 - 250 µm.

3. Revision PMMA bone cement according to claim 1 or 2, in which the granular antibiotics consist of one or more members of the group of gentamicin sulfate, gentamicin hydrochloride, amikacin sulfate, amikacin hydrochloride, tobramycin sulfate, tobramycin hydrochloride, clindamycin hydrochloride, lincosamine hydrochloride, moxifloxacin, ciprofloxacin, teicoplanin, vancomycin, ramoplanin, dalbavancin, daptomycin, tigecyclin, metronidazole, tinidazole, and omidazole.

4. Revision PMMA bone cement according to at least one of the preceding claims, whereby granular antibiotics from the group of the oxazolidones are used as granular antibiotics.

5. Revision PMMA bone cement according to at least one of the preceding claims, whereby the granular antibiotics component contains, in addition, at least one excipient from the group of polyvinylpyrrolidone, polyethylene glycol, polyethylene oxide, maltose, sorbitol, and mannitol.

6. Revision PMMA bone cement according to at least one of the preceding claims, whereby the granular antibiotics component contains, in addition, at least one excipient from the group of the toxicologically tolerable polymers, gelatine, collagen, and dextran.

7. Revision PMMA bone cement according to at least one of the preceding claims, whereby at least a fraction of the granular antibiotics is glued or grouted together by adhesive excipients to form antibiotic grains with particle sizes in the range of 63 - 900 µm.

## Revendications

1. Ciment osseux de révision en PMMA comprenant un composant pulvérulent et un composant liquide, **caractérisé en ce que** deux antibiotiques granuleux ou plus sont contenus dans le composant pulvérulent, lesquels coïncident au niveau de la distribution granulométrique **en ce que** la fraction granulométrique principale des antibiotiques individuels se situe à chaque fois dans une seule et même plage granulométrique, dans lequel la fraction granulométrique principale des antibiotiques comprend à chaque fois au moins 50 pour cent en poids de chaque antibiotique et dans lequel la plage granulométrique de la fraction granulométrique principale des antibiotiques individuels va de 100 à 250 µm.

2. Ciment osseux de révision en PMMA selon la revendication 1, **caractérisé en ce que** la plage granulométrique de la fraction granulométrique principale des antibiotiques individuels va de 150 à 250 µm.

3. Ciment osseux de révision en PMMA selon la revendication 1 ou 2, dans lequel les antibiotiques granuleux se composent d'un ou plusieurs membres du groupe du sulfate de gentamicine, du chlorhydrate de gentamicine, du sulfate d'amikacine, du chlorhydrate d'amikacine, du sulfate de tobramycine, du chlorhydrate de tobramycine, du chlorhydrate de clindamycine, du chlorhydrate de lincosamine, de la moxifloxacine, de la ciprofloxacine, de la teicoplanine, de la vancomycine, de la ramoplanine, de la dalbavancine, de la daptomycine, de la tigécycline, du métronidazole, du tinidazole et de l'omidazole.

4. Ciment osseux de révision en PMMA selon au moins une des revendications précédentes, dans lequel des antibiotiques du groupe des oxazolidones sont utilisés en tant qu'antibiotiques granuleux.

5. Ciment osseux de révision en PMMA selon au moins une des revendications précédentes, dans lequel le composant d'antibiotiques granuleux contient en outre au moins un adjuvant du groupe de la polyvinylpyrrolidone, du polyéthylèneglycol, de l'oxyde de polyéthylène, du maltose, du sorbitol et du mannitol.

6. Ciment osseux de révision en PMMA selon au moins une des revendications précédentes, dans lequel le composant d'antibiotiques granuleux contient en outre au moins un adjuvant du groupe des polymères acceptables du point de vue toxicologique que sont la gélatine, le collagène et le dextrane.

7. Ciment osseux de révision en PMMA selon au moins une des revendications précédentes, dans lequel au moins une partie des antibiotiques granuleux est collée ou cimentée d'adjuvants adhésifs en des granules d'antibiotiques avec des tailles particulaires dans la plage de 63 à 900 µm.
